# EUROPÄISCHE PATENTANMELDUNG

(11) **EP 0 800 840 A2**
(43) Veröffentlichungstag der Anmeldung: **15.10.1997**
(21) Anmeldenummer: 97103002.8
(22) Anmeldetag: 25.02.1997
(51) Int. Cl.: A61M 5/142, F04B 43/08

(54) **Infusionspumpe als Schlauchpumpe**

(30) Priorität: 14.03.1996 DE 29604737 U
(71) Anmelder: B. BRAUN MELSUNGEN AG, 34212 Melsungen (DE)
(72) Erfinder: Proell, Dieter, 34327 Körle (DE)
(74) Vertreter: Selting, Günther, Dipl.-Ing.

(57) **Zusammenfassung**

Die Infusionspumpe weist einen Schlauch (11) auf, der von Pumpenschiebern (12) fortlaufend flachgedrückt wird. Zur Rückformung des flachgedrückten Schlauchs in seine Ursprungsform ist eine Rückformeinrichtung (20) vorgesehen, die aus zwei zueinander gegenläufig bewegbaren Backen (21,22) besteht. Bei maximal zurückgezogenem Pumpenschieber (12) nimmt die Rückformeinrichtung (20) eine Schließstellung ein, wobei die Backen (21,22) den Schlauch in seine Ursprungs-Querschnittsform zurückformen. Ein darüber hinausgehendes Flachdrücken des Schlauchs erfolgt durch die Rückformeinrichtung nicht.

## Beschreibung

Die Erfindung betrifft eine Infusionspumpe als Schlauchpumpe, mit einem Schlauch und mehreren Pumpenschiebern, die den Schlauch aufeinanderfolgend und zyklisch zusammendrücken.

Eine Infusionspumpe dieser Art ist bekannt aus DE 40 35 182 C1. Diese Infusionspumpe weist zahlreiche Pumpenschieber auf, die einzeln von einer Nockenwelle angetrieben sind und den Schlauch gegen ein Widerlager drücken. Der Antrieb der Pumpenschieber erfolgt in der Weise, daß der Schlauch fortlaufend zusammengedrückt wird, so daß die in ihm enthaltene Flüssigkeit in Förderrichtung peristaltisch vorgetrieben wird. Solche Schlauchpumpen werden auch als Fingerpumpen bezeichnet, wobei die einzelnen Pumpenschieber die Finger bilden.

Es hat sich herausgestellt, daß die Schlauchpumpen bei längerer Betriebsdauer einen erheblichen Förderfehler haben. Daher können die Schlauchpumpen bei höheren Anforderungen an die Förderrate nicht benutzt werden.

Eine Infusionspumpe, von der der Oberbegriff des Patentanspruchs 1 ausgeht, ist bekannt aus DE 29 39 212 C2. Bei dieser Infusionspumpe ist ein von einer Nockenwelle gesteuerter linear bewegbarer Pumpenschieber vorgesehen, der den Schlauch abwechselnd flachdrückt und sich dann wieder zurückzieht. Der Pumpenschieber steuert eine aus zwei Backen bestehende Rückformeinrichtung, deren Backen seitlich an dem Schlauch angreifen. Die Rückformeinrichtung ist über die gesamte Zykluslänge gegenphasig zu dem Pumpenschieber gesteuert. Wenn der Pumpenschieber seine vordere Endstellung erreicht hat, befindet sich die Rückformeinrichtung in der Öffnungsstellung, in der sie ein Flachdrücken des Schlauches zuläßt. Wenn der Pumpenschieber dagegen seine maximale Rückzugsstellung erreicht hat, nimmt die Rückformeinrichtung eine Schließstellung ein, in der sie den Schlauch ihrerseits zusammendrückt, so daß der Schlauch völlig geschlossen wird. Nur auf der halben Länge des Vorschubwinkels des Pumpenschiebers nehmen der Pumpenschieber und die Rückformeinrichtung eine Stellung ein, in der der Schlauch seine normale Querschnittsform annehmen kann. Bei dieser Betriebsart wird der Schlauch abwechselnd in zwei rechtwinklig verlaufenden Richtungen vollständig zusammengequetscht. Er ist in jeder Phase des Pumpenzyklus externen Verformungskräften ausgesetzt. Da bei einer Schlauchpumpe zahlreiche Pumpenschieber mit relativ geringen Abständen zueinander angeordnet sind und alle Pumpenschieber sich in unterschiedlichen Phasen des Pumpenzyklus befinden, tritt der sogenannte Tetra-Effekt ein, bei dem der Schlauch auf unterschiedlichen Längenabschnitten gleichzeitig unterschiedlich zwangsgeformt wird. Dies kann zu Ermüdungen des Schlauchmaterials führen.

In der älteren Patentanmeldung WO 96/18038 (internationales Veröffentlichungsdatum: 13. Juni 1996) ist eine Schlauchpumpe beschrieben, bei der ebenfalls eine aus zwei Backen bestehende Rückformeinrichtung vorgesehen ist. Bei maximal zurückgezogenem Pumpenschieber greifen die Backen der Rückformeinrichtung an dem Schlauch an und stellen diesen auf seinen Ursprungsquerschnitt zurück. Bei der nachfolgenden Vorschubbewegung des Pumpenschiebers entfernen sich die Backen von dem Schlauch. Bei dieser Schlauchpumpe erstrecken sich die Backen der Rückformeinrichtung seitlich an der Nockenwelle vorbei über die Nockenwelle hinaus.

Der Erfindung liegt die Aufgabe zugrunde, eine Infusionspumpe in Form einer Schlauchpumpe oder Fingerpumpe zu schaffen, die eine erhöhte Förderkonstanz hat und den Schlauch nur gering belastet.

Die Lösung dieser Aufgabe erfolgt erfindungsgemäß mit den im Patentanspruch 1 angegebenen Merkmalen.

Bei der erfindungsgemäßen Infusionspumpe ist eine auf den Schlauch einwirkende Rückformeinrichtung vorgesehen, die bei zurückgezogenem Pumpenschieber den Schlauch quer zur Bewegungsrichtung des Pumpenschiebers zurückformt. Durch die Rückformeinrichtung wird das normale Relaxationsverhalten des Schlauchs unterstützt und der Schlauch schneller und genauer in seine Ursprungsform zurückversetzt. Damit werden Ermüdungserscheinungen des Schlauchmaterials eliminiert. Die Rückformeinrichtung sorgt dafür, daß der Schlauch beim nächsten Einwirken des Pumpenschiebers sich in seinem vorgesehenen Ursprungszustand befindet und den maximalen Schlauchquerschnitt präsentiert. Die Rückformeinrichtung verhindert das "Plattwalken" des Infusionsschlauchs bei längeren Infusionszeiten. Sie wird entsprechend der Position des zugehörigen Pumpenschiebers gesteuert. Vorzugsweise ist zu jedem Pumpenschieber eine zugehörige Rückformeinrichtung vorgesehen. Es kann jedoch ausreichen, wenn beispielsweise jedem zweiten Pumpenschieber eine Rückformeinrichtung zugeordnet ist.

Der Erfindung liegt die Erkenntnis zugrunde, daß die hohe Ungenauigkeit der Förderrate bei Schlauchpumpen auf das schlechte Relaxationsverhalten des Schlauchs zurückzuführen ist, der nach jedem Hub des Pumpenschiebers nicht mehr seine ursprüngliche Form einnimmt, sondern lediglich eine Zwischenform, bei der der Schlauchquerschnitt verringert ist. Durch die Rückformeinrichtung wird sichergestellt, daß nach jedem Hub des Pumpenschiebers der Schlauchquerschnitt seine Ursprungsform wieder einnimmt. Der Schlauch wird jedoch bei zurückgezogenem Pumpenschieber nur bis zum Erreichen seiner Ursprungs-Querschnittsform zurückgeformt und durch die Rückformeinrichtung nicht flachgedrückt. Außerdem greift die Rückformeinrichtung nur dann an dem Schlauch an, wenn dieser von dem Pumpenschieber freigegeben ist. Es erfolgt also keine ständige Zwangsverformung des Schlauches, so daß dieser wahrend des Pumpenzyklus phasenweise die Möglichkeit hat, sich frei einzustellen. Nur bei vollständig vorgeschobenem Pumpenschieber und in der Schließstellung der Rückformeinrichtung erfolgt jeweils eine Zwangsausübung auf den Schlauch. Beide Zwangsausübungen sind abwechselnd. Dies bedeutet, daß entweder nur der Pumpenschieber oder nur die Rückformeinrichtung an dem Schlauch angreift, aber zu keinem Zeitpunkt beide angreifen.

Bei einer bevorzugten Ausführungsform der Erfindung weist die Rückformeinrichtung zwei gegenläufig zueinander bewegbare Backen auf, deren einander zugewandte Arbeitsflächen dem Schlauchumfang des entsprechenden Schlauchs angepaßt sind. Durch Auswechseln oder Umstellung der Backen ist eine Anpassung an unterschiedliche Schlauchdurchmesser möglich.

Im folgenden werden unter Bezugnahme auf die Zeichnungen Ausführungsbeispiele der Erfindung näher erläutert.

Es zeigen:
- Fig. 1: einen Längsschnitt durch einen Teil der Schlauchpumpe bei einem ersten Ausführungsbeispiel,
- Fig. 2: einen schematischen Querschnitt entlang der Linie II-II von Fig. 1,
- Fig. 3: in gleicher Darstellung wie Fig. 2 den Zustand bei zurückgezogenem Pumpenschieber,
- Fig. 4: einen Querschnitt durch ein zweites Ausführungsbeispiel bei geöffneter Rückformeinrichtung, und
- Fig. 5: in gleicher Darstellung wie Fig. 4 die geöffnete Rückformeinrichtung.

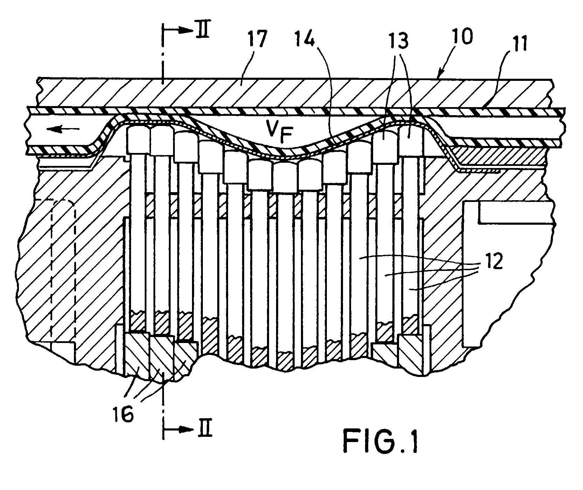

Die in Fig. 1 dargestellte Schlauchpumpe 10 weist einen flexiblen Schlauch 11 auf, der beispielsweise aus PVC besteht und im Ursprungszustand kreisrunden Querschnitt hat. Der Schlauch 11 ist ein Einmalartikel, der für den einmaligen Gebrauch in die Infusionspumpe eingesetzt und anschließend durch einen anderen Schlauch ersetzt wird. Der Schlauch 11 wird an eine Flüssigkeitsquelle, z.B. einen Infusionsflüssigkeitsbehälter, angeschlossen, um eine Infusionslösung über längere Zeit, z.B. 24 Stunden, einem Patienten mit möglichst gleichbleibender Infusionsrate zuzuführen.

Die Infusionspumpe 10 enthält zahlreiche parallele Pumpenschieber 12, deren Köpfe 13 über eine flexible Membran 14 an dem Schlauch 11 angreifen, so daß dieser entweder seine Ursprungsform annimmt oder zusammengedrückt wird. Die Membran 14 ist entbehrlich. Ein (nicht dargestellter) Antriebsmotor treibt eine in dem Pumpengehäuse gelagerte Nockenwelle 15, die zahlreiche Nockenscheiben 16 aufweist. Jede der Nockenscheiben 16 treibt einen Pumpenschieber 12. Die Pumpenschieber 12 sind in dem Pumpengehäuse in seitlichen Nuten längsverschiebbar geführt und sie verlaufen quer zur Längsrichtung des Schlauchs 11. Die Nockenscheiben 16 sind derart angeordnet, daß die Pumpenschieber 12 zyklisch und nacheinander vorgeschoben und zurückgezogen werden, wobei der Schlauch 11 über seine Länge etwa sinusförmig verformt wird und ein Fördervolumen V_{F} einschließt, das in Förderrichtung wandert. Auf der den Pumpenschiebern 12 entgegengesetzten Seite wird der Schlauch 11 durch eine Druckplatte 17 abgestützt.

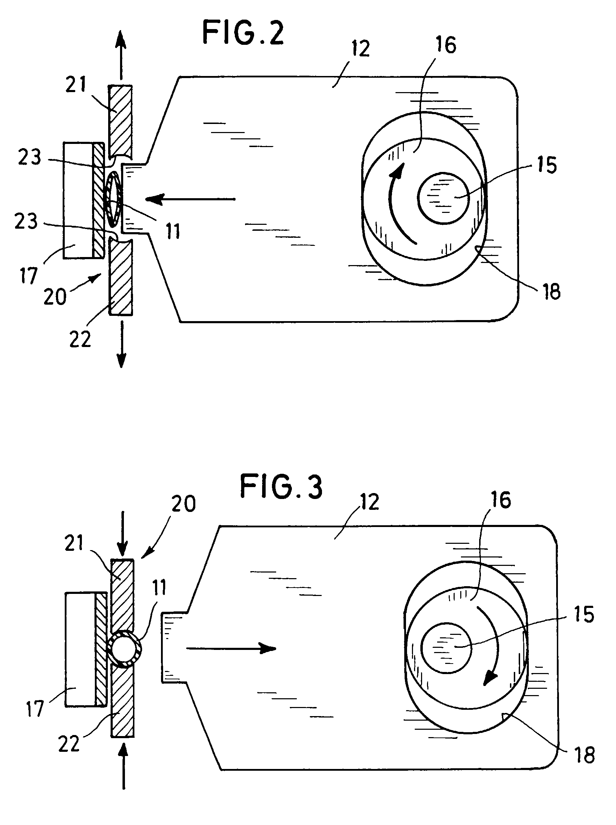

In den Fign. 2 und 3 ist einer der Pumpenschieber 12 in seiner Pumpposition (Fig. 2) und in seiner Rückzugsposition (Fig. 3) dargestellt. Die zugehörige Nockenscheibe 16, die auf der Nockenwelle 15 sitzt, greift in ein Langloch 18 des Pumpenschiebers 12 ein und bewegt den Pumpenschieber 12 quer zum Schlauch in Vorschubrichtung, um den Schlauch zusammenzudrücken, und anschließend in Rückzugsrichtung, um von dem Schlauch abzuheben.

Auf den Schlauch 11 wirkt eine Rückformeinrichtung 20 ein. Diese weist zwei Backen 21,22 auf, die in den angegebenen Pfeilrichtungen quer zur Längsrichtung des Schlauchs 11 und quer zur Bewegungsrichtung des Pumpenschiebers 12 hin- und herbewegt werden können, wobei die Bewegung der Backen 21,22 gegenläufig ist. In der Pumpposition (Fig. 2) sind die Backen 21,22 auseinanderbewegt, so daß sie den Schlauch 11 freigeben und dieser von dem Pumpenschieber 12 gegen die Druckplatte 17 flachgedrückt werden kann. In der Rückzugsposition (Fig. 3) des Pumpenschiebers 12 sind die Backen 21,22 gegeneinandergefahren, so daß sie von entgegengesetzten Seiten gegen den Schlauch drücken, jedoch einen solchen gegenseitigen Abstand einhalten, daß sie dessen Rundform wiederherstellen.

Die Backen 21,22 haben einander zugewandte Arbeitsflächen 23, deren Form dem Schlauchumfang des entspannten Schlauchs 11 angepaßt ist. Wie Fig. 3 zeigt, stoßen die Backen 21,22 während des Rückformvorganges nicht gegeneinander, sondern sie behalten einen derartigen Abstand, daß der Schlauch 11 seine Ursprungsform ungestört einnehmen kann.

Die Backen 21,22 sind synchron mit der Bewegung des zugehörigen Pumpenschiebers 12 gesteuert, wobei sie die Öffnungsposition (Fig. 2) einnehmen, wenn der Pumpenschieber in der Pumpposition ist, und die Schließposition (Fig. 3) einnehmen, wenn der Pumpenschieber 12 in der Rückzugsposition ist.

Vorzugsweise ist für jeden der Pumpenschieber 12 eine eigene Rückformeinrichtung 20 vorgesehen.

Versuche haben ergeben, daß mit der erfindungsgemäßen Infusionspumpe niedrige Förderfehler erreicht werden können.

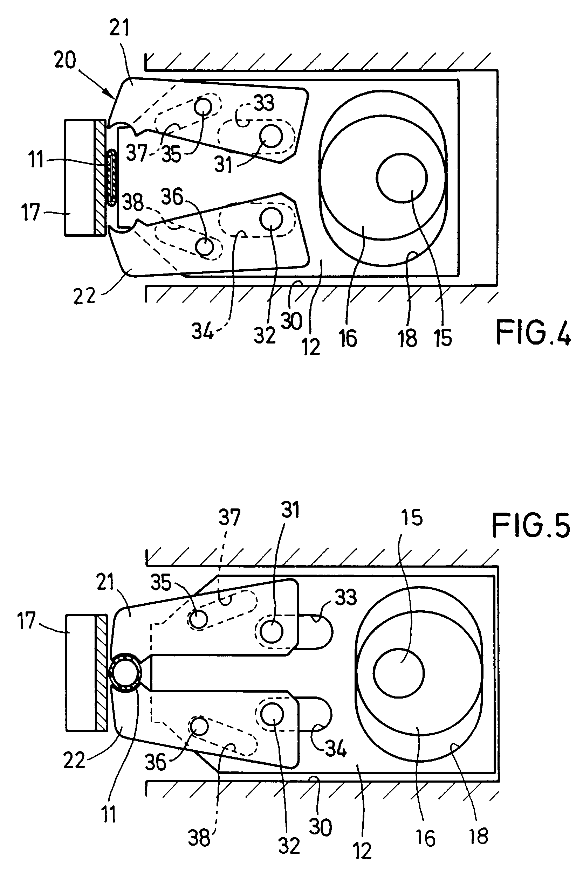

In den Fign. 4 und 5 ist ein weiteres Ausführungsbeispiel der Erfindung dargestellt. Der Pumpenschieber 12 wird hierbei ebenfalls von einer Nockenscheibe 16 angetrieben, die auf einer Nockenwelle 15 sitzt und in ein Langloch 18 des Pumpenschiebers 12 eingreift. Der Pumpenschieber 12 ist entlang einer Linearführung 30 linear quer zum Schlauch 11 geführt. An der Linearführung 30 ist für jede Backe 21,22 eine Achse 31 bzw. 32 vorgesehen, die ortsfest zu der Führung 30 angeordnet ist und um die die Backe 21 bzw. 22 geschwenkt werden kann. In dem Pumpenschieber 12 sind entsprechende Langlöcher 33,34 vorgesehen, durch die die Achsen 31,32 hindurchgehen, so daß die Bewegung des Pumpenschiebers durch die Achsen nicht gestört wird. Ferner sind an den Backen 21 und 22 Stifte 35,36 vorgesehen, die seitlich von jeder Backe abstehen. Diese Stifte 35,36 ragen in ein Langloch 37 bzw. 38 des Pumpenschiebers 12 hinein. Die Langlöcher 37,38 konvergieren zu dem schlauchseitigen Ende des Schiebers 12 hin. Sie bilden Kulissenführungen zur Steuerung der Schwenkbewegung der Schieber 21,22 um deren jeweilige Achse 31,32 herum. Die Backen 21,22 sind Platten, die parallel zu dem plattenförmigen Schieber 12 verlaufen.

In Fig. 4 ist der Pumpenschieber 12 in seiner Vorschubstellung dargestellt, in der er den Schlauch 11 gegen die Druckplatte 17 flachdrückt. Hierbei spreizen die Langlöcher 37,38 die Stifte 35 und 36 und mit diesen die Backen 21 und 22 auseinander, wobei die Backen 21,22 um ihre Achsen 31,32 herum verschwenkt werden, so daß sich die Rückformeinrichtung 20 in der Öffnungsstellung befindet.

In Fig. 5 ist der Zustand dargestellt, daß der Pumpenschieber 12 in der Rückzugsstellung ist. Hierbei steuern die Langlöcher 37,38 die Stifte 35 und 36 in der Weise, daß diese aufeinander zu bewegt werden, wodurch die Backen 21,22 in die Schließstellung gedrückt werden, in der sie den Schlauch 11 in seine Ursprungsstellung zurückformen.

## Patentansprüche

1. Infusionspumpe als Schlauchpumpe, mit einem Schlauch (11) und mehreren Pumpenschiebern (12), die von einer Nockenwelle (15) angetrieben sind und den Schlauch aufeinanderfolgend und zyklisch zusammendrücken, und mit einer aus zwei gegenläufig zueinander bewegbaren Backen (21,22) bestehenden Rückformeinrichtung, die bei maximal zurückgezogenem Pumpenschieber eine Schließstellung einnimmt,
**dadurch gekennzeichnet,**
daß die Rückformeinrichtung (20) derart ausgebildet ist, daß sie in der Schließstellung den Schlauch in seine normale Querschnittsgestalt mit maximalem Öffnungsquerschnitt zurückformt.

2. Infusionspumpe nach Anspruch 1, dadurch gekennzeichnet, daß die Backen (21,22) der Rückformeinrichtung (20) sich nur in demjenigen Bereich strecken, der zwischen der Nockenwelle (15) und dem Schlauch (11) liegt.

3. Infusionspumpe nach Anspruch 1 oder 2, dadurch gekennzeichnet, daß die Backen (21,22) an ihren einander zugewandten Arbeitsflächen (23) dem Schlauchumfang des entspannten Schlauches (11) angepaßt sind.

4. Infusionspumpe nach einem der Ansprüche 1-3, dadurch gekennzeichnet, daß die Backen (21,22) in einer parallel zu dem Pumpenschieber (12) verlaufenden Ebene schwenkbar und von dem Pumpenschieber (12) zwangsgesteuert sind.
